# EUROPÄISCHE PATENTSCHRIFT

(11) **EP 3 408 067 B1**
(45) Veröffentlichungstag und Bekanntmachung des Hinweises auf die Patenterteilung: **27.05.2020**
(21) Anmeldenummer: 17700593.1
(22) Anmeldetag: 11.01.2017
(51) Int. Cl.: B29C 65/08, A61F 13/15, A61F 13/62, A44B 18/00, B29C 65/78, B29L 31/48

(54) **VERFAHREN ZUM HERSTELLEN VON HYGIENEARTIKEL-VERSCHLUSSSYSTEMEN, INSBESONDERE WINDELVERSCHLUSS-SYSTEMEN**
METHOD FOR MANUFACTURING CLOSING SYSTEMS FOR HYGIENE ARTICLES, IN PARTICULAR DIAPER CLOSING SYSTEMS
PROCÉDÉ DE FABRICATION DE SYSTÈMES DE FERMETURE D'ARTICLES D'HYGIÈNE, EN PARTICULIER DE SYSTÈMES DE FERMETURE DE COUCHES

(30) Priorität: 25.01.2016 DE 102016000756
(43) Veröffentlichungstag der Anmeldung: 05.12.2018
(73) Patentinhaber: Gottlieb Binder GmbH & Co. KG, 71088 Holzgerlingen (DE)
(72) Erfinder: TUMA, Jan, 71083 Herrenberg (DE)
(74) Vertreter: Bartels, Martin Erich Arthur
(86) Internationale Anmeldenummer: PCT/EP2017/000029
(87) Internationale Veröffentlichungsnummer: WO 2017/129346

(56) Entgegenhaltungen:
- EP-A1- 2 815 733
- JP-A- 2011 015 796
- US-A1- 2003 235 660
- US-A1- 2004 116 889

## Beschreibung

Die Erfindung betrifft ein Verfahren zum Herstellen von Hygieneartikel-Verschlusssystemen, insbesondere Windelverschluss-Systemen, mit den Merkmalen im Oberbegriff des Anspruchs 1.

Für Einmalgebrauch vorgesehene Baby- oder Inkontinenzwindeln weisen Verschlusssysteme auf, die ein schnelles und bequemes Schließen und Öffnen der Windel ermöglichen und an der Windel seitlich angebrachte Festlegeteile aufweisen, die bandförmig sind oder in breiter Form sog. Windelohren bilden. Mit den Festlegeteilen verbundene Trägerteile weisen vorstehende Verschlusselemente auf, die beim Schließzustand des Verschlusssystems Haftverbindungen mit der Windel bilden. Bei der Herstellung dieser Verschlusssysteme, die aus mehreren Materiallagen, beispielsweise aus Nonwoven-Materialien, bestehen, ist es Stand der Technik, Verbindungsstellen zwischen den Lagen durch Klebeverbindungen oder durch Schweißverbindungen, wie Ultraschall-Schweißen, auszubilden. Der Ersatz von Klebeverbindungen durch Schweißverbindungen, wie Ultraschall-Schweißen, wie es in dem Dokument EP 2 564 822 B1 für das Herstellen von Verbindungen zwischen Kunststofffolien von Windelverschlusssystemen aufgezeigt ist, hat gegenüber der Bildung von Klebeverbindungen unter anderem den Vorteil, dass benutzte Windeln problemlos entsorgbar sind, weil keine die biologische Abbaubarkeit gefährdenden Klebstoffmaterialien vorhanden sind, wobei auch die Gefahr einer Gesundheitsschädigung des Windelträgers aufgrund einer Toxizität des Klebstoffes vermieden ist, was insbesondere bei Babywindeln einen besonderen Vorteil darstellt.

Der Einsatz des Ultraschall-Schweißverfahrens ist bei Verschlusssystemen, bei denen ein Trägerteil, das mit Verschlusselementen versehen ist, mit einem Festlegeteil zu verbinden ist, insofern problematisch, weil die im Schweißbereich stattfindende thermische Einwirkung auf die vorstehenden Haftverschlusselemente zu deren Deformation im Schweißbereich führt, womit ein Verlust an Haftkraft einhergeht, der die Sicherheit des mittels des Verschlusselements gebildeten Windelverschlusses beeinträchtigt. Um den Verlust an Haftkraft gering zu halten, ist man daher gezwungen, lediglich punktuell Schweißstellen auszubilden, was zum einen die sichere Verbindung zwischen Trägerteil und Festlegeteil beeinträchtigt und zum anderen fertigungstechnisch aufwendig ist.

Die EP 2 815 733 A1 offenbart ein Verfahren zum Herstellen von Hygieneartikel-Verschlusssystemen, wobei einem Trägerteil mit vorstehenden Verschlusselementen, die in Reihen mit vorgebbarem Abstand zueinander angeordnet sind, auf dessen Rückseite ein Festlegeteil zugeführt und mittels eines Ultraschall-Schweißverfahrens mit dem Trägerteil entlang von Schweißstellen verbunden wird, wobei zumindest ein Teil der Schweißstellen entlang von Schweißverbindungslinien zwischen benachbarten Reihen von Verschlusselementen und zumindest teilweise parallel zu diesen verlaufend eingebracht wird, wobei mindestens ein Ambossteil einer Ultraschall-Schweißanlage zwecks Erhalt der jeweiligen Schweißverbindungslinie zwischen die Abstände benachbart angeordneter Reihen von Verschlusselementen und parallel zu diesen Reihen verlaufend in Eingriff gebracht wird, und wobei die mit dem jeweiligen Ambossteil zusammenwirkende Sonotrode oder Sonotrodenteil in Anlage mit derjenigen Seite des Festlegeteiles kommt, die den Verschlusselementen abgewandt ist.

Die US 2004/0116889 und die JP 2011-15796 offenbaren weitere Hygieneartikel-Verschlusssysteme.

Ausgehend von diesem Stand der Technik stellt sich die Erfindung die Aufgabe, ein Verfahren zum Herstellen von Hygieneartikel-Verschlusssystemen anzugeben, das die sichere Verbindung von Trägerteil und Festlegeteil durch Ultraschall-Schweißen ohne Gefährdung der Schließeigenschaft des Verschluss-Systems ermöglicht.

Erfindungsgemäß ist diese Aufgabe durch ein Verfahren gelöst, das die Merkmale des Patentanspruchs 1 in seiner Gesamtheit aufweist.

Gemäß dem kennzeichnenden Teil des Anspruchs 1 zeichnet sich das erfindungsgemäße Verfahren dadurch aus, dass die jeweiligen Ambossteile als Bestandteil eines Blockwerkzeugs eingesetzt werden, dass mehrere, auf vorgebbarem Abstand in Anpassung an den Reihenabstand zwischen den Verschlusselementen des Trägerteils zueinander gehaltene Ambossteile parallel zueinander verlaufend Teile des Blockwerkzeugs bilden, und dass für den Erhalt einer Führung für das Trägerteil, das für einen Schweißvorgang zusammen mit dem Festlegeteil entlang des Blockwerkzeuges verfahren wird, am Blockwerkzeug als Führungsleisten dienende Ambossteile verwendet werden, die in die Abstände zwischen den benachbarten Reihen von Verschlusselementen, vorzugsweise mit teilweise Untergreifen derselben, eingreifen.

Durch die mittels der Ambossteile zusätzlich gebildete Führung ist ein besonders sicherer Schweißvorgang ohne Gefährdung der Verschlusselemente durchführbar.

Ferner besteht eine wesentliche Besonderheit der Erfindung darin, dass zumindest ein Teil der Schweißstellen entlang von Schweißverbindungslinien zwischen benachbarten Reihen von Verschlusselementen und zumindest teilweise parallel zu diesen verlaufend eingebracht wird. Dadurch, dass die Schweißstellen zwischen benachbarten Reihen von Verschlusselementen verlaufen und dadurch die thermische Einwirkung auf die Zwischenbereiche zwischen den Verschlusselementen begrenzt ist, ist zum einen der Verlust an Haftkraft vermieden. Durch zu den Reihen parallel verlaufende Schweißverbindungslinien lässt sich zum anderen eine verhältnismäßig großflächige Schweißverbindung herstellen, so dass auch eine sichere Verankerung zwischen Trägerteil und Festlegeteil gewährleistet ist. Mindestens ein Ambossteil einer Ultraschall-Schweißanlage wird zwecks Erhalt der jeweiligen Schweißverbindungslinie zwischen die Abstände benachbart angeordneter Reihen von Verschlusselementen und parallel zu diesen verlaufend in Eingriff gebracht und die mit dem jeweiligen Ambossteil zusammenwirkende Sonotrode kommt in Anlage mit derjenigen Seite des Festlegeteils, die den Verschlusselementen abgewandt ist.

Die jeweiligen Ambossteile werden als Bestandteil eines Blockgwerkzeuges eingesetzt, wobei mehrere, auf vorgebbarem Abstand in Anpassung an den Reihenabstand zwischen den Verschlusselementen des Trägerteils zueinander gehaltene Ambossteile parallel zueinander verlaufend Teile des Block- oder Scheibenwerkzeugs bilden. Dadurch lassen sich auf besonders rationelle Weise durch Relativbewegung zwischen den Materialbahnen und dem Blockwerkzeug gleichzeitig eine Mehrzahl von Schweißverbindungslinien und damit ein großflächiger Verbindungsbereich ausbilden.

Für den Erhalt einer Führung für das Trägerteil, das für einen Schweißvorgang zusammen mit dem Festlegeteil entlang eines vorzugsweise drehbar angeordneten nicht erfindungsgemäßen Scheibenwerkzeugs verfahren wird, kann mit Vorteil auch derart vorgegangen werden, dass in Verfahrrichtung gesehen vor und/oder hinter dem Scheibenwerkzeug Führungsteile angeordnet werden, die die Reihen von Verschlusselementen positionieren zwecks definiertem Eingriff des jeweiligen Ambossteils des Scheibenwerkzeugs zwischen die zuordenbaren Reihen von Verschlusselementen.

Vorzugsweise werden die Verschlusselemente des Trägerteils sowohl in Längs- als auch in Querrichtung in Reihen angeordnet, wobei der Abstand zwischen den Verschlusselementen sowohl in Längs- als auch in Querrichtung gleich gehalten wird.

Die jeweiligen Schweißverbindungslinien zwischen Träger- und Festlegeteil werden jeweils derart eingebracht, dass die Verschlusselemente des Trägerteils frei von Beschädigungen mit dem Festlegeteil verschweißt werden.

Hinsichtlich der Ausbildung der einen Haftverschluss bildenden Verschlusselemente kann so vorgegangen werden, dass neben Verschlusshaken oder Schlaufen besonders bevorzugt Verschlussköpfe zum Einsatz kommen, die mittels Stielteilen auf der Oberseite des Trägerteils vorstehen und zumindest teilweise über die Stielteile endseitig unter Bildung einer Verhakungsmöglichkeit für korrespondierend ausgebildete Verschlusselemente eines weiteren Trägerteils vorstehend ausgebildet werden.

Wie in dem Dokument DE 196 46 318 A1 offenbart, kann das Trägerteil mit seinen Verschlusselementen einstückig ausgebildet werden, wobei das Festlegeteil aus einem Nonwoven gebildet wird und wobei für beide Teile thermoplastische Kunststoffmaterialien eingesetzt werden, wie Polyolefine oder Polyamid (PA12).

Das Festlegeteil kann zumindest teilweise über das angeschweißte Trägerteil mit den Verschlusselementen randseitig vorstehend ausgebildet werden, wobei das jeweils vorstehende Teil des Festlegeteils zumindest teilweise als Anbindungsteil für den betreffenden Hygieneartikel, wie eine Baby- oder Inkontinenzwindel, genutzt wird. Das dem Anbindungsteil entgegengesetzte vorstehende Teil des Festlegeteils kann als Grifflasche für ein bequemes Öffnen des betreffenden Windelverschlusses genutzt werden.

Gegenstand der Offenbarung ist auch ein Anbindungsteil, insbesondere für einen Hygieneartikel, wie eine Baby- oder Inkontinenzwindel, mit einem Trägerteil mit vorstehenden, Reihen bildenden Verschlusselementen, das mit einem Festlegeteil mittels Schweißverbindungsstellen verschweißt ist, wobei die Schweißverbindungsstellen zumindest teilweise linienförmig und parallel zwischen benachbart angeordneten Reihen von Verschlusselementen des Trägerteils verlaufen.

Gegenstand der Offenbarung ist auch ein Hygieneartikel, wie eine Baby- oder Inkontinenzwindel, der zumindest ein Anbindungsteil aufweist, wobei das Anbindungsteil, vorzugsweise jedoch zwei Anbindungsteile, dem wahlweisen Öffnen und Schließen des Hygieneartikels dienen.

Nachstehend ist die Erfindung anhand von in der Zeichnung dargestellten Ausführungsbeispielen im Einzelnen erläutert. Es zeigen:
- Fig. 1: eine schematisierte Draufsicht einer Babywindel mit im geöffneten Zustand gezeichnetem, nach dem erfindungsgemäßen Verfahren hergestelltem Verschlusssystem;
- Fig. 2: eine in etwa 50-facher Vergrößerung gezeichnete Teildraufsicht des Trägerteils des Verschlusssystems;
- Fig. 3: eine schematisch vereinfacht gezeichnete perspektivische Schrägansicht einer Ultraschall-Schweißeinrichtung zur Durchführung des erfindungsgemäßen Verfahrens zur Herstellung des Verschlusssystems;
- Fig. 4: einen vergrößert gezeichneten Teilausschnitt des in Fig. 3 mit IV bezeichneten Bezirks einer Ultraschall-Schweißeinrichtung zur Durchführung des erfindungsgemäßen Herstellverfahrens;
- Fig. 5: eine schematisch vereinfacht gezeichnete perspektivische Schrägansicht einer weiteren Ultraschall-Schweißeinrichtung zur Durchführung eines nicht erfindungsgemäßen Herstellverfahrens; und
- Fig. 6: einen vergrößert gezeichneten Teilausschnitt des in Fig. 5 mit VI bezeichneten Bezirks der Schweißeinrichtung.

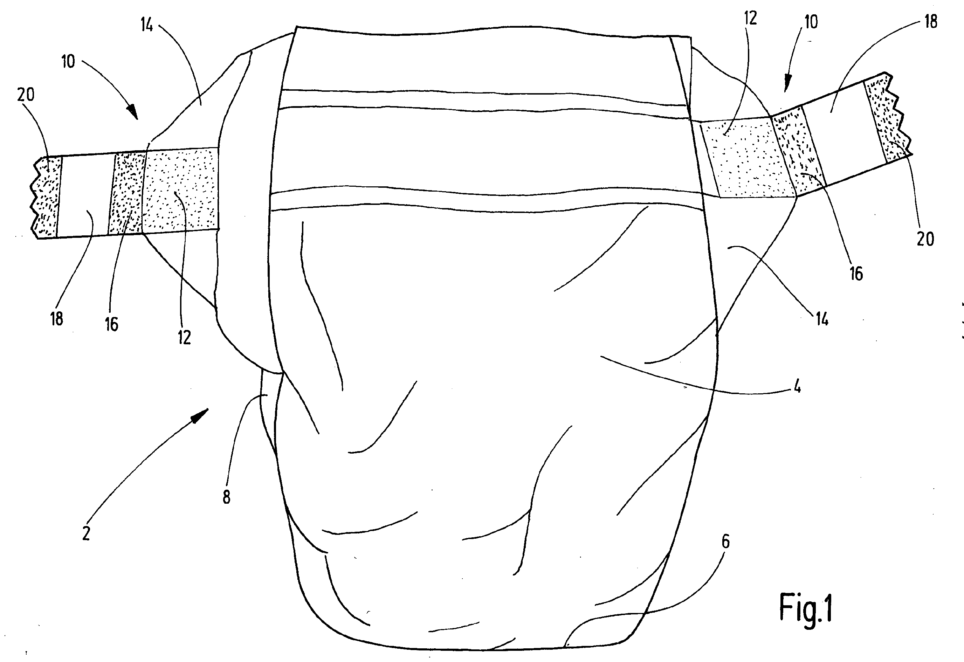

Die Fig. 1 zeigt eine für Einmalgebrauch bestimmte Babywindel 2 mit Blickrichtung auf die Windelvorderseite 4, die um den in Fig. 1 untenliegenden Randbereich 6 umgelegt ist und auf der Windelrückseite 8 aufliegt. Das Verschlusssystem, das in Fig. 1 in geöffnetem Zustand dargestellt ist, weist Anbindungsteile 10 auf, die jedes durch den freiliegenden Abschnitt eines bandförmigen Festlegeteils 16 gebildet sind, die mit ihrem Endabschnitt 12 an einem Ansatz 14 angebracht sind, die sich in Form sogenannter Windelohren an beiden Seiten der Windelrückseite 8 befinden. Die Festlegeteile 16 sind aus einem Nonwoven aus thermoplastischem Kunststoffmaterial, wie einem Polyolefin, Polyester oder Polyamid (PA12), gebildet und die Ansätze 14 bestehen ebenfalls aus einem entsprechenden, gute Schweißeigenschaften aufweisenden thermoplastischen Material, so dass die Verbindung der Endabschnitte 12 mit den Ansätzen 14 in vorteilhafter Weise durch Verschweißen gebildet ist.

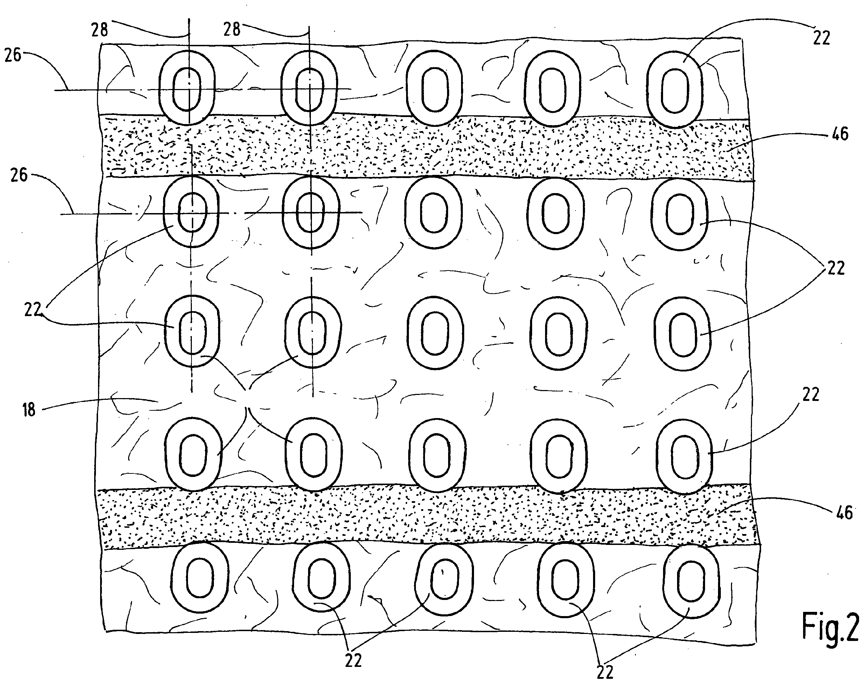

Auf dem über den jeweiligen Ansatz 14 vorstehenden Abschnitt des Festlegeteils 16 befindet sich ein mit vorstehenden Verschlusselementen versehenes Trägerteil 18 in einem Abstand vom am freien Ende befindlichen Abschnitt 20, der eine vom Benutzer ergreifbare Grifflasche bildet. Das Trägerteil18 weist an der Oberseite vorstehende Verschlusselemente, die lediglich in Fig. 2, 4 und 6 sichtbar und dort teilweise mit 22 beziffert sind, auf, die beim Schließzustand des Verschlusssystems, wenn die Anbindungsteile 10 auf die Windelvorderseite 4 umgelegt sind, mit dieser als einem weiteren Trägerteil eine Haftverbindung bilden, deren Haftkraft das Verschlusssystem im Schließzustand sichert.

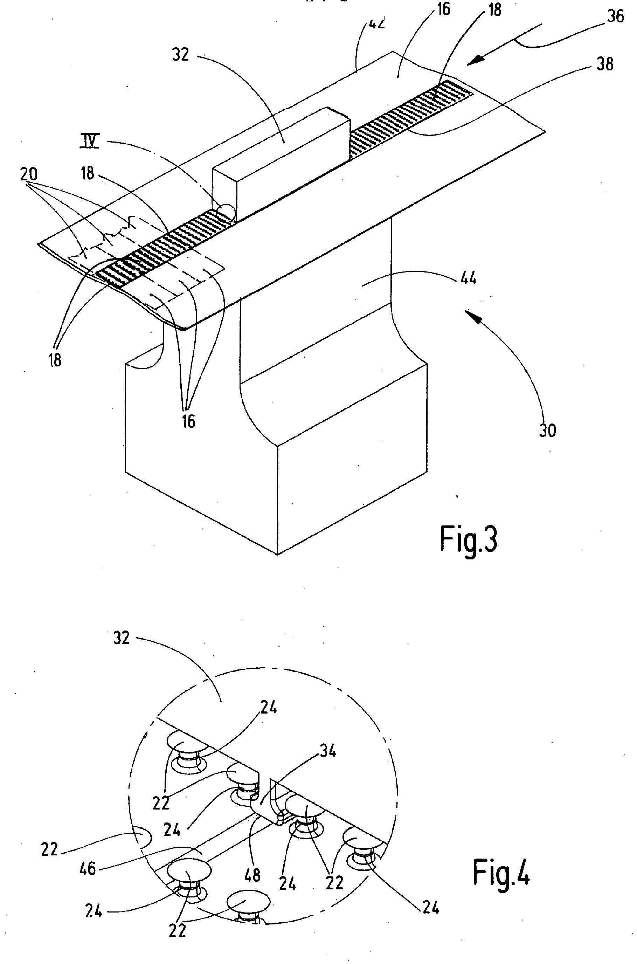

Wie die Fig. 2 und 4 am deutlichsten zeigen, weisen die Verschlusselemente 22 Verschlussköpfe auf, die pilzkopfartig geformt und über Stielteile 24 (Fig. 4) einstückig mit dem Nonwoven des Trägerteils 18 verbunden sind. Die Verschlussköpfe können rund, oval oder mehreckig, insbesondere sechseckig ausgebildet sein. Beispielsweise kann das Trägerteil 18 aus einem Polyolefin, Polyester oder aus Polyamid (PA12) zusammen mit den Verschluss-elementen 22 in einem Arbeitsgang mittels einer Einrichtung, wie sie im Dokument DE 196 46 318 A1 offenbart ist, in einem kontinuierlichen Verfahren hergestellt werden, indem das thermoplastische Kunststoffmaterial dem Spalt zwischen einer Druckwalze und einer Formwalze zugeführt wird, die ein Sieb mit offenen Hohlräumen aufweist, die den Verschluss-elementen 22 ihre Form vermitteln und dabei die Verschlusselemente in geradlinigen Reihen von Zeilen 26 und dazu senkrechten Spalten 28 anordnen, wie dies in Fig. 2 gezeigt ist. Wie dieser Figur ebenfalls entnehmbar ist, haben die Verschlusselemente 22 in Richtung der Zeilen 26 jeweils den gleichen Abstand voneinander, der den gegenseitigen Abständen, gesehen in Richtung der Spalten 28, entspricht, so dass das in Fig. 2 gezeigte regelmäßige Anordnungsmuster der Verschlusselemente 22 gebildet ist.

Die Fig. 3 und 4 zeigen eine erste Ausführungsform des erfindungsgemäßen Herstellverfahrens. Für die Ausbildung der Schweißverbindung zwischen Trägerteil 18 und Festlegeteil 16 wird eine Ultraschall-Schweißanlage 30 eingesetzt, die in einem Blockwerkzeug 32 befindliche Ambossteile 34 aufweist, von denen lediglich eine in Fig. 4 sichtbar ist. In einer in Fig. 3 mit Pfeil 36 bezeichneten Vorschubrichtung wird als Halbfertigteil, von dem nach erfolgtem Schweißvorgang die einzelnen Anbindungsteile 10 abgetrennt werden, eine unabgeteilte Bahn 42 des Festlegeteils 16 mit daraufliegendem Streifen 38 des vorgefertigten Trägerteils 18 unterhalb der Ambossteile 34 des Blockwerkzeugs 32 zwischen diesem und einer Sonotrode 44 hindurchgeführt, deren Sonotrodenfläche sich über die in Vorschubrichtung gemessene Länge des Blockwerkzeugs 32 erstreckt. Die Ambossteile 34, die sich ebenfalls über diese Länge des Blockwerkzeugs 32 erstrecken, sind in zur Vorschubrichtung senkrecht verlaufender Richtung in solchen Abständen voneinander angeordnet, dass sie nach jeweils drei Zeilen 26 (Fig. 2) der Verschlusselemente 22 auf eine in Vorschubrichtung verlaufende Reihe ausgerichtet sind, so dass die in Fig. 2 mit 46 bezeichneten Schweißverbindungslinien gebildet werden. Wie in Fig. 3 mit strichpunktierten Linien angedeutet ist, werden nach erfolgtem Schweißvorgang die fertiggestellten Anbindungsteile 10 von der Bahn 42 abgetrennt.

Die genaue Positionierung der Schweißverbindungslinien 46 im Zwischenraum zwischen benachbarten Verschlusselementen 22, wodurch deren thermische Deformation beim Schweißvorgang verhindert ist, erfolgt bei dieser Ausführungsform durch von den Ambossteilen 34 ausgeübte Führungsfunktion, die sich als Führungsleisten entlang der gesamten, in Vorschubrichtung gemessene Länge des Blockwerkzeugs 32 erstrecken. Wie die Fig. 4 zeigt, weisen die Ambossteile 34 für ihre Führungsfunktion einen Querschnitt in Form eines kopfstehenden T auf, dessen endseitiger Querbalken 48 die Köpfe benachbarter Reihen der Verschlusselemente 22 untergreift, so dass die Querbalken 48 seitliche Führungsflächen für die Stiele 24 der Verschlusselemente 22 der zugeordneten Reihen bilden.

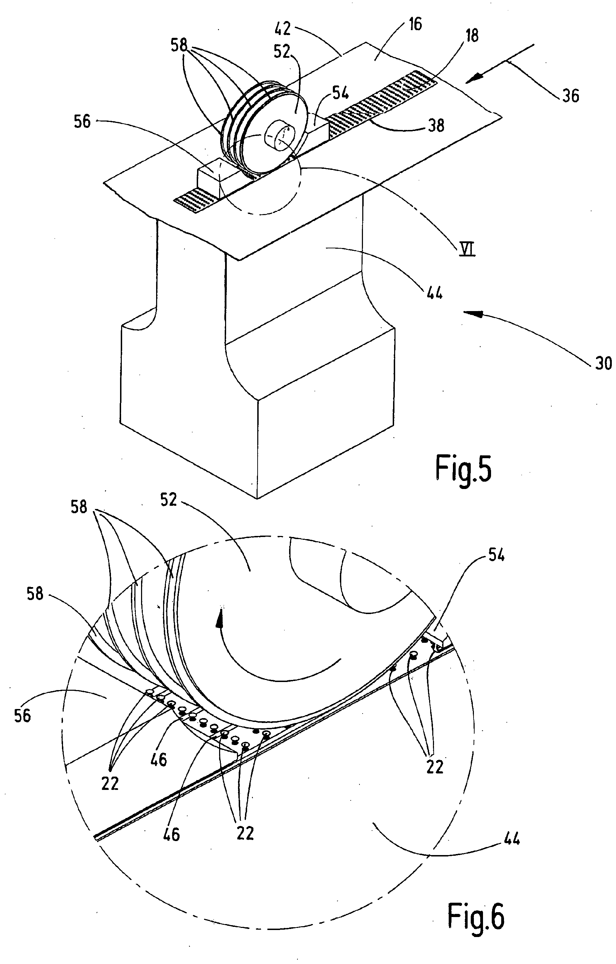

Bei der Ausführungsform von Fig. 5 und 6 kommt eine nicht erfindungsgemäße Ultraschall-Schweißanlage 30 zum Einsatz, bei der bei sonst gleichem Anlagenaufbau anstelle eines die Ambossteile 34 aufweisenden Blockwerkzeugs 32 ein Scheibenwerkzeug 52 benutzt wird und wobei, in Vorschubrichtung gesehen unmittelbar vor und hinter dem Scheibenwerkzeug 52, Führungsteile 54 und 56 angeordnet sind, die die Führungsfunktion übernehmen, die bei der Schweißanlage 30 von Fig. 3 und 4 durch die Ambossteile 34 ausgeübt wird. Diese Führungsteile 54 und 56 weisen an der in der Zeichnung nicht sichtbaren Unterseite vorstehende Führungsrippen auf, die in entsprechender Weise, wie es in Fig. 4 für die Ambossteile 34 gezeigt ist, die Führungsfunktion durch Untergriff der Köpfe an den Stielen 24 benachbarter Reihen von Verschlusselementen 22 ausüben.

Am Außenumfang des Scheibenwerkzeugs 52 sind Ambossteile 58 durch radial vorstehende, in Umfangsrichtung durchgehend verlaufende Rippen vorgesehen, die bei der Drehbewegung des Scheibenwerkzeugs 52, die bei der Vorschubbewegung der Bahn 42 und des Streifens 38 erfolgt, zwischen einer betreffenden Reihe benachbarter Verschlusselemente 22 abrollen und dabei die Schweißverbindungslinien 46 zusammen mit der Flächen-Sonotrode 44 in gleicher Weise erzeugen wie bei der Ausführungsform von Fig. 3 und 4. Mit dem jeweiligen ringförmigen Ambossteil 58, das drehend um seine Längsachse ausgebildet sein kann, lassen sich aufgrund der kleineren Angriffsfläche mit dem zu verschweißenden Medium gegenüber dem Blockwerkzeug 32 sehr hohe Anpress- und Schweißkräfte verwirklichen. Auch ist die Rotationsgeschwindigkeit des Scheibenwerkzeuges 52 als Amboss für die Sonotrode 44 an die Vorschubgeschwindigkeit von Bahn 42 und Streifen 38 angepasst.

Die Ausbildung der Schweißverbindungslinien 46 in Richtung von Zeilen 26 (s. Fig. 2), die in der Vorschubrichtung der betreffenden Ultraschall-Schweißanlage 30 verlaufen, hat den Vorteil, dass der Schweißvorgang in kontinuierlichem Vorschub erfolgen kann, was eine besonders rationelle Fertigung der Anbindungsteile 10 ermöglicht. Alternativ könnten jedoch auch, gegebenenfalls bei diskontinuierlich erfolgendem Schweißvorgang, Schweißverbindungslinien in Richtung der Streifen 28 oder abwechselnd in den beiden Richtungen ausgeführt werden. Während bei den in den Figuren gezeigten Beispielen Verschlusselemente 22 mit auf Stielen 24 befindlichen Pilzköpfen vorgesehen sind, versteht sich, dass bei entsprechend ausgebildeten, die Haftverbindung mit den Trägerteilen 18 bildenden weiteren Trägerteilen, die sich beispielsweise an der Windelvorderseite 4 befinden, andersartig geformte Verschlusselemente, wie Haken oder Schlaufen, benutzt werden können.

Es liegt noch im Bereich der Offenbarung, das jeweilige Ambossteil 34 oder 58 als Sonotrode auszugestalten und die Sonotrode 44 als Amboss der Ultraschall-Schweißanlage 30 zu betreiben.

## Patentansprüche

1. Verfahren zum Herstellen von Hygieneartikel-Verschlusssystemen, insbesondere Windelverschluss-Systemen, wobei einem Trägerteil (18) mit vorstehenden Verschlusselementen (22), die in Reihen (26, 28) mit vorgebbarem Abstand zueinander angeordnet sind, auf dessen Rückseite ein Festlegeteil (16) zugeführt und mittels eines Ultraschall-Schweißverfahrens mit dem Trägerteil (18) entlang von Schweißstellen verbunden wird, wobei zumindest ein Teil der Schweißstellen entlang von Schweißverbindungslinien (46) zwischen benachbarten Reihen (26) von Verschlusselementen (22) und zumindest teilweise parallel zu diesen verlaufend eingebracht wird, wobei mindestens ein Ambossteil (34) einer Ultraschall-Schweißanlage (30) zwecks Erhalt der jeweiligen Schweißverbindungslinie (46) zwischen die Abstände benachbart angeordneter Reihen (26) von Verschlusselementen (22) und parallel zu diesen Reihen (26) verlaufend in Eingriff gebracht wird, und wobei die mit dem jeweiligen Ambossteil (34) zusammenwirkende Sonotrode (44) oder Sonotrodenteil in Anlage mit derjenigen Seite des Festlegeteiles (16) kommt, die den Verschlusselementen (22) abgewandt ist, **dadurch gekennzeichnet, dass** die jeweiligen Ambossteile (34) als Bestandteil eines Blockwerkzeugs (32) eingesetzt werden, dass mehrere, auf vorgebbarem Abstand in Anpassung an den Reihenabstand zwischen den Verschlusselementen (22) des Trägerteils (18) zueinander gehaltene Ambossteile (34) parallel zueinander verlaufend Teile des Blockwerkzeugs (32) bilden, und dass für den Erhalt einer Führung für das Trägerteil (18), das für einen Schweißvorgang zusammen mit dem Festlegeteil (16) entlang des Blockwerkzeuges (32) verfahren wird, am Blockwerkzeug (32) als Führungsleisten dienende Ambossteile (34) verwendet werden, die in die Abstände zwischen den benachbarten Reihen (26) von Verschlusselementen (22) eingreifen.

2. Verfahren nach Anspruch 1, **dadurch gekennzeichnet, dass** Ambossteile (34) in die Abstände zwischen den benachbarten Reihen (26) von Verschlusselementen (22) mit teilweisem Untergreifen derselben eingreifen.

3. Verfahren nach einem der vorstehenden Ansprüche, **dadurch gekennzeichnet, dass** die Verschlusselemente (22) des Trägerteils (18) sowohl in Längs- als auch in Querrichtung in Reihen (26, 28) angeordnet werden und dass der Abstand zwischen den Verschlusselementen (22) sowohl in Längs- als auch in Querrichtung gleich gehalten wird.

4. Verfahren nach einem der vorstehenden Ansprüche, **dadurch gekennzeichnet, dass** die jeweiligen Schweißverbindungslinien (46) zwischen Träger- (18) und Festlegeteil (16) derart eingebracht werden, dass die Verschlusselemente (22) des Trägerteils (18) frei von Beschädigungen mit dem Festlegeteil (16) verschweißt werden.

5. Verfahren nach einem der vorstehenden Ansprüche, **dadurch gekennzeichnet, dass** als Verschlusselemente neben Verschlusshaken oder Schlaufen besonders bevorzugt Verschlussköpfe (22) zum Einsatz kommen, die mittels Stielteilen (24) auf der Oberseite des Trägerteiles (18) vorstehen und zumindest teilweise über die Stielteile (24) endseitig unter Bildung einer Verhakungsmöglichkeit für korrespondierend ausgebildete Verschlusselemente eines weiteren Trägerteils (4) vorstehend ausgebildet werden.

6. Verfahren nach einem der vorstehenden Ansprüche, **dadurch gekennzeichnet, dass** das Trägerteil (18) mit seinen Verschlusselementen (22) einstückig ausgebildet wird, dass das Festlegeteil (16) aus einem Non-woven gebildet wird und dass für beide Teile (16, 18) thermoplastische Kunststoffmaterialien eingesetzt werden, wie Polyolefine, Polyester oder Polyamid (PA12).

7. Verfahren nach einem der vorstehenden Ansprüche, **dadurch gekennzeichnet, dass** das Festlegeteil (16) zumindest teilweise über das angeschweißte Trägerteil (18) mit den Verschlusselementen (22) randseitig vorstehend ausgebildet wird und dass das jeweils vorstehende Teil (12, 20) des Festlegeteils zumindest teilweise als Anbindungsteil (10) für einen Hygieneartikel, wie eine Baby- (2) oder Inkontinenzwindel, genutzt wird.

## Claims

1. Method for producing closing systems for hygiene articles, in particular nappy closing systems, wherein a fixing element (16) is applied to the rear side of a supporting element (18) with protruding fastening elements (22), which are arranged in rows (26, 28) at a pre-definable distance from one another, and joined to said supporting element (18) by means of an ultrasonic bonding process along bonding points, at least some of the bonding points being applied along bonding lines (46) between adjacent rows (26) of fastening elements (22) and running at least partially parallel to these rows, wherein at least one anvil part (34) of an ultrasonic bonding device (30) is engaged between the rows (26) of fastening elements (22) arranged adjacent to spaces and running parallel to these rows (26) for the purpose of obtaining the respective bonding line (46), and wherein the sonotrode (44) or sonotrode part interacting with the respective anvil part (34) comes into contact with the side of the fixing element (16) which faces away from the fastening elements (22), **characterised in that** the respective anvil parts (34) are used as constituent parts of a block die (32), **in that** a plurality of anvil parts (34) held a pre-definable distance from each other to adapt to the row spacing between the fastening elements (22) of the supporting element (18) form parts of the block die (32) running parallel to one another, and **in that**, to obtain a guide for the supporting element (18), which is moved together with the fixing element (16) along the block die (32) for a bonding process, anvil parts (34) acting as guide strips are used on the block die (32), said anvil parts engaging in the spaces between the adjacent rows (26) of fastening elements (22).

2. Method according to claim 1, **characterised in that** anvil parts (34) engage in the spaces between the adjacent rows (26) of fastening elements (22), gripping beneath said fastening elements in some cases.

3. Method according to any one of the preceding claims, **characterised in that** the fastening elements (22) of the supporting element (18) are arranged in rows (26, 28) in both the longitudinal and transverse direction and **in that** the space between the fastening elements (22) is kept the same in both the longitudinal and transverse direction.

4. Method according to any one of the preceding claims, **characterised in that** the respective bonding lines (46) between the supporting (18) and fixing element (16) are applied such that the fastening elements (22) of the supporting element (18) are bonded to the fixing element (16) without any damage.

5. Method according to any one of the preceding claims, **characterised in that**, in addition to fastening hooks or loops, fastening heads (22) are used more preferably as fastening elements which protrude on the upper side of the supporting element (18) by means of stem parts (24) and **in that** an additional supporting element (4) is designed to protrude at least partially above the stem parts (24) at the end, providing an interlocking opportunity for fastening elements with a corresponding shape.

6. Method according to any one of the preceding claims, **characterised in that** the supporting element (18) is designed in one piece with its fastening elements (22), **in that** the fixing element (16) is made from a non-woven material and **in that** thermoplastic plastics materials such as polyolefins, polyester or polyamide (PA12) are used for both elements (16, 18).

7. Method according to any one of the preceding claims, **characterised in that** the fixing part (16) is formed such that it protrudes at least partially at its edges above the bonded supporting element (18) with the fastening elements (22) and **in that** the respective protruding part (12, 20) of the fixing element is used at least partially as a joining element (10) for a hygiene article, such as a nappy (2) or incontinence pants.

## Revendications

1. Procédé de fabrication de systèmes de fermeture d'articles d'hygiène, notamment de systèmes de fermeture de couche, dans lequel on met, sur une partie (18) formant support et ayant des éléments (22) de fermeture en saillie, disposés en rangées (26, 28) à une distance pouvant être donnée à l'avance les unes des autres sur sa face arrière, une partie (16) de fixation et on la relie le long de points de soudure à la partie (18) formant support au moyen d'un procédé de soudure par ultrasons, au moins une partie des points de soudure étant mise entre des rangées (26) voisines d'éléments (22) de fermeture et s'étendant, au moins en partie, parallèlement à celles-ci, au moins une partie (34) d'enclume d'une installation (30) de soudure par ultrasons intervenant en s'étendant, en vue d'obtenir les lignes (46) de liaison de soudure respectives, entre les intervalles entre des rangées (26) voisines d'éléments (22) de fermeture et parallèlement à ces rangées (26), et la sonotrode (44) ou la partie formant sonotrode coopérant avec la partie (34) d'enclume respective venant en contact avec la face de la partie (16) de fixation, qui est loin des éléments (22) de fermeture, **caractérisé en ce que** l'on utilise les parties (34) respectives d'enclume comme parties constitutives d'un outil-bloc (32), **en ce que** plusieurs parties (34) d'enclume, maintenues entre elles à une distance pouvant être donnée à l'avance en adaptation à la distance dans la rangée entre les éléments (22) de fermeture de la partie (18) de support, forment des parties s'étendant parallèlement entre elles de l'outil-bloc (32) et **en ce que**, pour obtenir un guidage de la partie (18) de support, qui, pour une opération de soudure, est déplacée ensemble avec la partie (16) de fixation le long de l'outil-bloc (32), on utilise des parties (34) d'enclume, qui servent à l'outil-bloc (32) de réglettes de guidage et qui pénètrent dans les intervalles entre les rangées (26) voisines d'éléments (22) de fermeture.

2. Procédé suivant la revendication 1, **caractérisé en ce que** des parties (34) d'enclume pénètrent dans les intervalles entre les rangées (26) voisines d'éléments (22) de fermeture en prenant ceux-ci en partie par le dessous.

3. Procédé suivant l'une des revendications précédentes, **caractérisé en ce que** les éléments (22) de fermeture de la partie (18) de support sont disposés tant dans la direction longitudinale, qu'également dans la direction transversale, en rangées (26, 28) et **en ce que** l'intervalle entre les éléments (22) de fermeture est maintenu égal tant dans la direction longitudinale, qu'également dans la direction transversale.

4. Procédé suivant l'une des revendications précédentes, **caractérisé en ce que** l'on met les lignes (46) de liaison de soudure respective entre la partie (18) de support et la partie (16) de fixation, de manière à ce que les éléments (22) de fermeture de la partie (18) de support soient soudés à la partie (16) de fixation sans endommagement.

5. Procédé suivant l'une des revendications précédentes, **caractérisé en ce que** l'on met en œuvre, comme éléments de fermeture, outre des crochets de fermeture ou des boucles, d'une manière particulièrement préférée, des têtes (22) de fermeture, qui, au moyen de parties (24) de tige, font saillie de la face supérieure de la partie (18) de support et qui sont constituées en saillie, au moins partiellement par les parties (24) de tige du côté de l'extrémité, avec formation d'une possibilité d'accrochage d'éléments de fermeture constitués de manière correspondante d'une autre partie (4) de support.

6. Procédé suivant l'une des revendications précédentes, **caractérisé en ce que** la partie (18) de support est constituée d'une seule pièce avec ses éléments (22) de fermeture, **en ce que** la partie (16) de fixation est formée d'un non-tissé et **en ce que** l'on utilise, pour les deux parties (16, 18), des matières plastiques thermoplastiques, comme des polyoléfines, du polyester ou du polyamide (PA12).

7. Procédé suivant l'une des revendications précédentes, **caractérisé en ce que** la partie (16) de fixation est constituée en saillie du côté du bord, au moins partiellement par la partie (18) de support soudée ayant les éléments (22) de fermeture, et **en ce que** la partie (12, 20) en saillie respective de la partie de fixation est utilisée, au moins partiellement, comme partie d'attache d'une couche de bébé (2) ou d'incontinence.
